# EUROPEAN PATENT SPECIFICATION

(11) **EP 1 741 693 B1**
(45) Date of publication and mention of the grant of the patent: **03.08.2011**
(21) Application number: 04724173.2
(22) Date of filing: 29.03.2004
(51) Int. Cl.: C07C 29/145, C07C 33/30, C07C 33/46, C07C 31/20, C07C 35/32, C07C 39/11, C07C 43/23, C07D 311/22, C07D 495/04, C07B 53/00, B01J 31/22, B01J 31/18, B01J 31/24

(54) **PROCESS FOR PRODUCTION OF OPTICALLY ACTIVE ALCOHOLS**
VERFAHREN ZUR HERSTELLUNG VON OPTISCH AKTIVEN ALKOHOLEN
PROCEDE DE FABRICATION D' ALCOOLS OPTIQUEMENT ACTIFS

(43) Date of publication of application: 10.01.2007
(73) Proprietor: Nagoya Industrial Science Research Institute, Nagoya-shi, Aichi 460-0008 (JP); Kanto Kagaku Kabushiki Kaisha, Chuo-ku, Tokyo 103-0023 (JP)
(72) Inventor: NOYORI, Ryoji, Chiyoda-ku, Tokyo 102-0081 (JP); OHKUMA, Takeshi, Kita-11-jou, Chuou-ku, Sapporo-shi, Hokkaido 060-0011 (JP); TSUTSUMI, Kunihiko, Tokyo 187-0021 (JP); UTSUMI, Noriyuki, Soka-shi, Saitama 3400014 (JP); MURATA, Kunihiko, Koshigaya-shi, Saitama 3430838 (JP)
(74) Representative: Cloughley, Peter Andrew
(86) International application number: PCT/JP2004/004422
(87) International publication number: WO 2005/092825

(56) References cited:
- EP-A- 1 209 150
- EP-A- 1 254 885
- WO-A-02/055195
- JP-A- 11 189 600
- JP-A- 11 322 649
- JP-A- 2003 104 993
- RYOJI NOYORI AND SHOHEI HASHIGUCHI: "ASYMMETRIC TRANSFER HYDROGENATION CATALYZED BY CHIRAL RUTHENIUM COMPLEXES" ACCOUNTS OF CHEMICAL RESEARCH, vol. 30, no. 2, 1997, pages 97-102, XP002474775
- KAMALUDDIN ABDUR-RASHID ET AL: 'Mechanism of the hydrogenation of ketones catalyzed by trans-dihydrido(diamine)ruthenium(II) complexes' JOURNAL OF THE AMERICAN SOCIETY vol. 124, no. 50, 2002, pages 15104 - 15118, XP002980284
- KAMALUDDIN ABDUR-RASHID ET AL.: 'Catalytic cycle for the asymmetric hydrogenation of prochiral ketones to chiral alcohols: Direct hydride and proton transfer from chiral catalysts trans-Ru(H)2(diphosphine)(diamine) to ketones and direct addition of dihydrogen to the resulting hydridoamido complexes' JOURNAL OF THE AMERICAN CHEMICAL SOCIETY vol. 123, no. 30, 2001, pages 7473 - 7474, XP002980285

## Description

### Technical Field

The present invention relates to a process for producing an optically active alcohol in the presence of a catalyst such as a ruthenium metal complex.

### Background Art

Various processes for producing optically active alcohols in the presence of metal complexes as catalysts have been reported to date. In particular, a process for synthesizing an optically active alcohol from a carbonyl compound in the presence of an asymmetric metal complex as a catalyst has been intently studied.

For example, Japanese Unexamined Patent Application Publication No. 2003-104993 reports several examples of producing optically active alcohols by hydrogenation of various ketone compounds in 2-propanol without addition of any base under pressurized hydrogen catalyzed by a tetrahydroborate of an asymmetric ruthenium metal complex that has a diamine compound and a diphosphine compound, such as BINAP (2,2'-bis(diphenylphosphino)-1,1'-binaphthyl) or the like, coordinated to ruthenium. In particular, corresponding optically active alcohols are produced from acetophenone, ethyl 4-acetylbenzoate, and 3-nonen-2-one.

Japanese Unexamined Patent Application Publication No. Hei11-322649 reports an example of producing a corresponding optically active alcohol by hydrogenation of m-trifluoromethylacetophenone in the presence of triethylamine and an azeotrope of formic acid and triethylamine while using as a catalyst an asymmetric ruthenium metal complex in which a diphenylethylenediamine having a sulfonyl group on the nitrogen and a benzene derivative are coordinated to ruthenium.

However, when the catalyst disclosed in Japanese Unexamined Patent Application Publication No. 2003-104993 is used, although an optically active alcohol can be produced from a ketone compound without any base, the yield or the enantiomeric excess is low for some reaction substrates. In Japanese Unexamined Patent Application Publication No. Hei11-322649 , since triethylamine, which is an organic base, is necessary, production of an optically active alcohol from a reaction substrate, such as acetylene ketone which is unstable in the presence of bases, has been difficult.

European Patent Application published as EP 1209150 describes a process for the preparation of tricyclic amino alcohol derivatives. The process comprises the steps of: reducing a compound having a first formula, to yield a halohydrin having a second formula; converting the halohydrin under alkaline conditions into an epoxy compound having a third formula; reacting the epoxy compound with a compound having a fourth formula, to yield an amino alcohol having a fifth formula; reducing the resulting nitro group to give an aniline derivative having a sixth formula; reacting the aniline derivative with a sulfonating agent to give an amino alcohol having a seventh formula; finally, simultaneously or sequentially removing the protecting groups to yield the required compound.

### Disclosure of Invention

The present invention has been made to overcome the problems described above. An object of the present invention is to provide a process for producing an optically active alcohol from a ketone compound, hydrogenation of which has been difficult, in high yield and with high stereoselectivity.

To overcome these problems, the present inventors have studied the catalytic activity of many asymmetric ruthenium, rhodium, and iridium complexes, analyzed the principles of catalytic action, and developed a process of obtaining an optically active alcohol from a ketone compound, hydrogenation of which has been difficult, in high yield and with high stereoselectivity based on extensive studies.

The present invention provides a first process for producing an optically active alcohol, including placing a metal complex represented by formula (1) and a ketone compound in a polar solvent and stirring the mixture under pressurized hydrogen to hydrogenate the ketone compound and to thereby produce the optically active alcohol:

### General Formula (1)

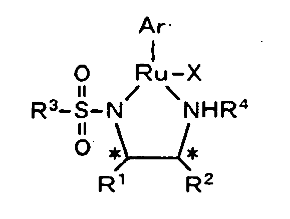

(where R¹ and R² may be the same or different and are each selected from the group consisting of an alkyl group, an optionally substituted phenyl group, an optionally substituted naphthyl group, and an optionally substituted cycloalkyl group, or together form an optionally substituted alicyclic ring;
R³ is one selected from the group consisting of an alkyl group, a perfluoroalkyl group, an optionally substituted naphthyl group, an optionally substituted phenyl group, and a camphor group;
R⁴ is a hydrogen atom or an alkyl group;
Ar is an optionally substituted benzene;
X is an anionic group; and
* represents an asymmetric carbon.)

According to this first process, since hydrogenation of a ketone compound proceeds under pressurized hydrogen, an optically active alcohol can be obtained from the ketone compound, hydrogenation of which has been difficult, in high yield and with high stereoselectivity.

The present invention also provides a second process for producing an optically active alcohol including placing a metal complex represented by formula (2) and a ketone compound in a polar solvent and stirring the mixture under pressurized hydrogen to hydrogenate the ketone compound and to thereby produce the optically active alcohol:

### General Formula (2)

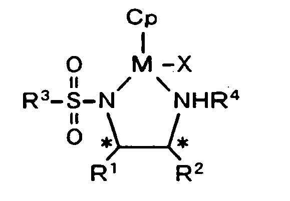

(where R¹ and R² may be the same or different and are each selected from the group consisting of an alkyl group, an optionally substituted phenyl group, an optionally substituted naphthyl group, and an optionally substituted cycloalkyl group, or together form an optionally substituted alicyclic ring;
R³ is one selected from the group consisting of an alkyl group, a perfluoroalkyl group, an optionally substituted naphthyl group, an optionally substituted phenyl group, and a camphor group;
R⁴ is a hydrogen atom or an alkyl group;
Cp is an optionally substituted cyclopentadiene;
M is rhodium or iridium;
X is an anionic group; and
* represents an asymmetric carbon.)
Also according to this second process, since
hydrogenation of a ketone compound proceeds under pressurized hydrogen, an optically active alcohol can be obtained from the ketone compound, hydrogenation of which has been difficult, in high yield and with high stereoselectivity.

Examples of the alkyl group for R¹ and R² of general formula (1) or (2) include C₁-C₁₀ alkyl groups, such as a methyl group, an ethyl group, an n-propyl group, an isopropyl group, an n-butyl group, a sec-butyl group, and a tert-butyl group. Examples of the optionally substituted phenyl group include an unsubstituted phenyl group, an alkyl-containing phenyl group, such as a 4-methylphenyl group or a 3,5-dimethylphenyl group, a phenyl group having a halogen substituent, such as a 4-fluorophenyl group or a 4-chlorophenyl group, and an alkoxy-containing phenyl group, such as a 4-methoxyphenyl group. Examples of the optionally substituted naphthyl group include an unsubstituted naphthyl group, a 5,6,7,8-tetrahydro-1-naphthyl group, and a 5,6,7,8-tetrahydro-2-naphthyl group. Examples of the optionally substituted cycloalkyl group include a cyclopentyl group and a cyclohexyl group. Examples of the substituted or unsubstituted alicyclic ring formed by R¹ and R² include a cyclohexane ring formed by R¹ and R². R¹ and R² are preferably both a phenyl group or preferably form a cyclohexane ring by binding each other.

Examples of the alkyl group for R³ in general formula (1) or (2) include C₁-C₁₀ alkyl groups such as a methyl group, an ethyl group, an n-propyl group, an isopropyl group, an n-butyl group, a sec-butyl group, and a tert-butyl group. Examples of the perfluoroalkyl group include a trifluoromethyl group and a pentafluoroethyl group. Examples of the optionally substituted naphthyl group include an unsubstituted naphthyl group, a 5,6,7,8-tetrahydro-1-naphthyl group, and a 5,6,7,8-tetrahydro-2-naphthyl group. Examples of the optionally substituted phenyl group include an unsubstituted phenyl group, an alkyl-containing phenyl group, such as a 4-methylphenyl group, a 3,5-dimethylphenyl group, a 2,4,6-trimethylphenyl group, and a 2,4,6-triisopropylphenyl group, a phenyl group having a halogen substituent, such as a 4-fluorophenyl group or a 4-chlorophenyl group, and an alkoxy-containing phenyl group, such as a 4-methoxyphenyl group.

Examples of the alkyl group for R⁴ in general formula (1) or (2) include a methyl group and an ethyl group. R⁴ is preferably hydrogen.

Examples of Ar in general formula (1) include in addition to unsubstituted benzene, alkyl-containing benzene such as toluene, o-, m-, or p-xylene, o-, m-, or p-cymene, 1,2,3-, 1,2,4-, or 1,3,5-trimethylbenzene, 1,2,4,5- or 1,2,3,4-tetramethylbenzene, pentamethylbenzene, and hexamethylbenzene.

Examples of Cp in general formula (2) include, in addition to unsubstituted cyclopentadiene, alkyl-containing cyclopentadiene such as mono-, di-, tri-, tetra-, or pentamethylcyclopentadiene.

X in general formula (1) or (2) is an anionic group. Examples thereof include a fluorine group, a chlorine group, a bromine group, an iodine group, a tetrafluoroborate group, a tetrahydroborate group, a
tetrakis[3,5-bis(trifluoromethyl)phenyl]borate group, an acetoxy group, a benzoyloxy group, a 2,6-dihydroxybenzoyl)oxy group, a (2,5-dihydroxybenzoyl)oxy group, a (3-aminobenzoyl)oxy group, a (2,6-methoxybenzoyl)oxy group, a (2,4,6-triisopropylbenzoyl)oxy group, a
1-naphthalenecarboxylic acid group, 2-naphthalenecarboxylic acid group, a trifluoroacetoxy group, a
trifluoromethanesulfoxy group, and a
trifluoromethanesulfonimide group. X is preferably a halogen group such as a fluorine group, a chlorine group, a bromine group, or an iodine group.
R¹, R², and R³ in general formula (1) or (2) may be the same or different and each preferably represent a phenyl group, a phenyl group containing a C₁-C₅ alkyl group, a phenyl group containing a C₁-C₅ alkoxy group, or a phenyl group containing a halogen substituent; and R⁴ is preferably a hydrogen atom. Since a bidentate ligand, an ethylene diamine derivative (R³SO₂NHCHR¹CHR²NHR⁴), is coordinated to ruthenium in general formula (1) and to rhodium or iridium in general formula (2), specific preferable examples of R¹ to R⁴ are described below as the examples of ethylenediamine derivatives. Namely, examples of the ethylenediamine derivatives include TsDPEN (N-(p-toluenesulfonyl)-1,2-diphenylethylenediamine, MsDPEN (N-methanesulfonyl-1,2-diphenylethylenediamine, N-methyl-N'-(p-toluenesulfonyl)-1,2-diphenylethylenediamin e, N-(p-methoxyphenylsulfonyl)-1,2-diphenylethylenediamine, N-(p-chlorophenylsulfonyl)-1,2-diphenylethylenediamine, N-trifluoromethanesulfonyl-1,2-diphenylethylenediamine, N-(2,4,6-trimethylbenzenesulfonyl)-1,2-diphenylethylenedia mine,
N-(2,4,6-triisopropylbenzenesulfonyl)-1,2-diphenylethylene diamine,
N-(4-tert-butylbenzenesulfonyl)-1,2-diphenylethylenediamin e, N-(2-naphthylsulfonyl)-1,2-diphenylethylenediamine, N-(3,5-dimethylbenzenesulfonyl)-1,2-diphenylethylenediamin e,N-pentamethylbenzenesulfonyl-1,2-diphenylethylenediamine, and 1,2-N-tosylcyclohexanediamine.

The metal complexes represented by general formulae (1) and (2) may each include one or more coordinating organic solvents. Examples of the coordinating organic solvent include aromatic hydrocarbon solvents such as toluene and xylene, aliphatic hydrocarbon solvents such as pentane and hexane, halogen-containing hydrocarbon solvents such as methylene chloride, ether solvents such as ether and tetrahydrofuran, alcohol solvents such as methanol, ethanol, 2-propanol, butanol, and benzyl alcohol, ketone solvents such as acetone, methyl ethyl ketone, and cyclohexyl ketone, and heteroatom-containing organic solvents such as acetonitrile, dimethylformamide (DMF), N-methylpyrrolidone, dimethyl sulfoxide (DMSO), and triethylamine.

Methods for preparing ruthenium, rhodium, and iridium complexes represented by general formulae (1) and (2) are disclosed in Angew. Chem., Int. Ed. Engl. Vol. 36, p. 285 (1997), J. Org. Chem. Vol. 64, p. 2186 (1999). That is, synthesis is possible through reaction between a sulfonyl diamine ligand and a ruthenium, rhodium, or iridium complex having a ligand X. Alternatively, synthesis is possible through reaction between HX and a metal amide complex having a sulfonyl diamine ligand.

Examples of the ruthenium complex which is used as the starting material for the ruthenium complex represented by general formula (1) include inorganic ruthenium compounds such as ruthenium(III) chloride hydrate, ruthenium(III) bromide hydrate, and ruthenium(III) iodide hydrate; dieneliganded ruthenium compounds, such as a [ruthenium dichloride(norbornadiene)] polynuclear complex, a [ruthenium dichloride(cycloocta-1,5-diene)] polynuclear complex, and bis(methylallyl)ruthenium(cycloocta-1,5-diene); aromatic-compound-liganded ruthenium complexes such as a [ruthenium dichloride(benzene)] polynuclear complex, a

[ruthenium dichloride(p-cymene)] polynuclear complex, a [ruthenium dichloride(trimethylbenzene)] polynuclear complex, and a [ruthenium dichloride(hexamethylbenzene)] polynuclear complex; phosphine-liganded complexes such as dichlorotris(triphenylphosphine)ruthenium; ruthenium dichloride(dimetfiylformamide)₄; and chlorohydridetris(triphenylphosphine)ruthenium. The ruthenium complex may be any other ruthenium complex that has a ligand substitutable with an optically active diphosphine compound and an optically active diamine compound and is not limited to those described above. For example, various ruthenium complexes disclosed in COMPREHENSIVE ORGANOMETALLIC CHEMISTRY II Vol. 7, pp. 294-296 (PERGAMON) can be used as the starting material.

Examples of the rhodium and iridium complexes that can be used as the starting materials for the asymmetric rhodium complex and the asymmetric iridium complex represented by general formula (2) include inorganic ruthenium compounds such as rhodium (III) chloride hydrate, rhodium (III) bromide hydrate, and rhodium(III) iodide hydrate, a [pentamethylcyclopentadienylrhodium dichloride] polynuclear complex, a [pentamethylcyclopentadienylrhodium dibromide] polynuclear complex, and a [pentamethylcyclopentadienylrhodium diiodide] polynuclear complex.

The reaction between the starting materials, i.e., the ruthenium, rhodium, and iridium complexes, and the ligands are carried out in at least one solvent selected from the group consisting of aromatic hydrocarbon solvents such as toluene and xylene, aliphatic hydrocarbon solvents such as pentane and hexane, halogen-containing hydrocarbon solvents such as methylene chloride, ether solvents such as ether and tetrahydrofuran, alcohol solvents such as methanol, ethanol, 2-propanol, butanol, and benzyl alcohol, and heteroatom-containing organic solvents such as acetonitrile, DMF, N-methylpyrrolidone, and DMSO, at a reaction temperature of 0°C to 200°C to thereby yield a metal complex.

According to each of the first and second processes of the present invention, a metal complex represented by general formula (1) or (2) and ketone are placed in a polar solvent and mixed under pressurized hydrogen to hydrogenate the ketone compounds. The pressure of the hydrogen is more preferably 5 to 150 atm from the standpoint of economy. The reaction can be carried out in the range of -50°C to 100°C, preferably in the range of -30°C to 50°C, and most preferably in the range of 20°C to 50°C. The reaction time differs depending on reaction conditions including the reaction substrate concentration, temperature and pressure. Typically, the reaction is completed in several minutes to several days and frequently in 5 to 24 hours. The purification of the reaction product can be conducted by a known method such as column chromatography, distillation, or recrystallization. In using the metal complex represented by general formula (1) or (2), an amide complex corresponding to the metal complex represented by general formula (1) or (2) may be added (e.g., metal complex:amide complex = 1.0:0 to 1.0 molar equivalent), or, HX (X as defined above) may be added to the metal complex represented by general formula (1) or (2) (e.g., metal complex:HX = 1.0:0 to 0.5 molar equivalents). Furthermore, hydrogenation reaction of the ketone compound may be carried out in the reaction system after preparation of the metal catalyst represented by general formula (1) or (2) from the corresponding amide complex and HX (e.g., amide complex:HX = 1.0:0.5 to 1.5 molar equivalents).

Examples of the polar solvent used in the first and second processes of the present invention include alcohol solvents such as methanol, ethanol, 2-propanol, 2-methyl-2-propanol, and 2-methyl-2-butanol, ether solvents such as tetrahydrofuran (THF) and diethyl ether, and heteroatom-containing solvents such as DMSO, DMF, and acetonitrile. These solvents may be used alone or in combination. A mixed solvent containing the polar solvent above and a solvent other than those described above may also be used. Among these polar solvents, alcohol solvents are preferable, methanol and ethanol are more preferable, and methanol is most preferable.

The amounts of the metal complexes represented by general formulae (1) and (2) used in the first and second processes of the present invention are preferably in the range of S/C of 10 to 100,000 and more preferably in the range of S/C of 50 to 10,000, where S/C is a molar ratio of the ketone compound to the metal complex, S representing the substrate and C representing the catalyst.

In the reaction systems according to the first and second processes of the present invention, a salt of an organic or inorganic substance may be added if necessary. Examples of the salt include ionic salts such as lithium perchlorate, sodium perchlorate, magnesium perchlorate, barium perchlorate, calcium perchlorate, lithium hexafluorophosphate, sodium hexafluorophosphate, magnesium hexafluorophosphate, calcium hexafluorophosphate, lithium tetrafluoroborate, sodium tetrafluoroborate, magnesium tetrafluoroborate, calcium tetrafluoroborate, lithium tetraphenylborate, sodium tetraphenylborate, magnesium tetraphenylborate, and calcium tetraphenylborate. To the metal complex, 1 to 1,000 molar equivalents of the salt may be added to hydrogenate the ketone. Preferably, 10 to 200 molar equivalents of a perchlorate is used relative to the metal complex.

The asymmetric carbons in the metal complexes represented by general formulae (1) and (2) of the first and second processes of the present invention can be obtained as either the (R) isomer or the (S) isomer. By selecting either one of the (R) and (S) isomers, the target (R) or (S) isomer of an optically active alcohol can be obtained at high selectivity.

According to the first and second processes of the present invention, it is not essential to add a base to the reaction system. Hydrogenation reaction of the ketone compound rapidly proceeds without addition of the base. However, this should not be taken to exclude addition of the base; for example, a small amount of a base may be added depending on the reaction substrate.

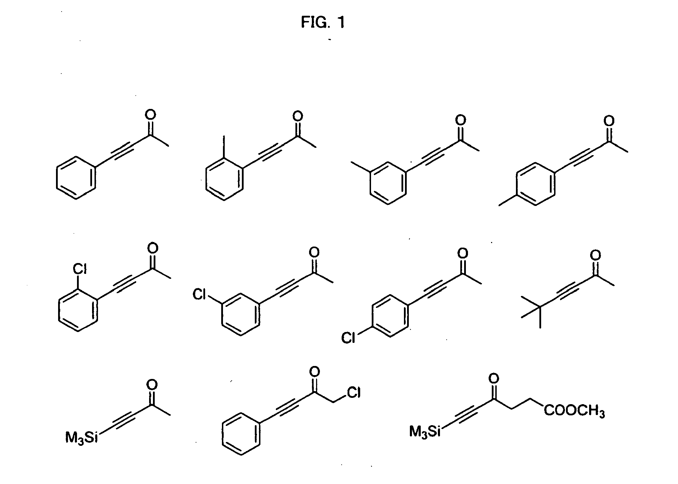

As described above, the processes for producing an optically active alcohol according to the first and second processes of the present invention do not essentially require a base to conduct hydrogenation of the ketone compound. Thus, a ketone compound that is unstable in the presence of bases can be hydrogenated to obtain a corresponding optically active alcohol. In particular, a cyclic ketone can be hydrogenated to give an optically active cyclic alcohol; a ketone having an olefin moiety or an acetylene moiety (especially a ketone in which the α,β-bond is the olefin moiety or acetylene moiety) can be hydrogenated to give an optically active alcohol having an olefin moiety or an acetylene moiety; a ketone having a hydroxyl group can be hydrogenated to give an optically active alcohol having a hydroxyl group; a ketone having a halogen substituent (especially a ketone having a halogen substituent at the α-position) can be hydrogenated to give an optically active alcohol having a halogen substituent; a chromanone derivative can be hydrogenated to give an optically active chromanol; a diketone can be hydrogenated to give an optically active diol; a ketoester can be hydrogenated to give an optically active hydroxy ester; and a ketoamide can be hydrogenated to give an optically active hydroxyamide. The representative examples of the ketone compounds applicable to the first process of the present invention are shown in Figs. 1 to 7.

### Brief Description of the Drawings

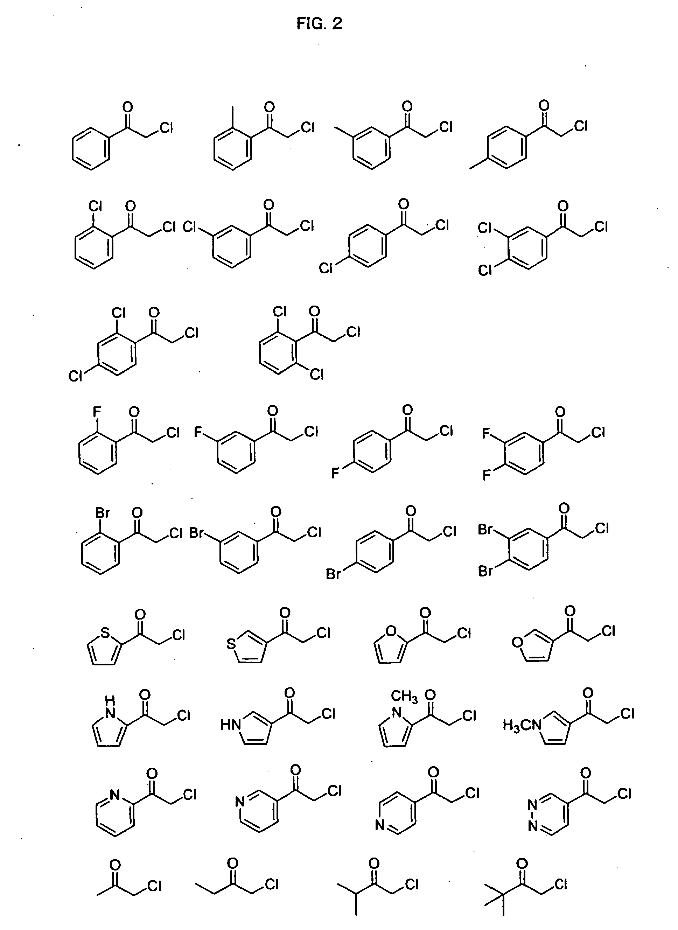

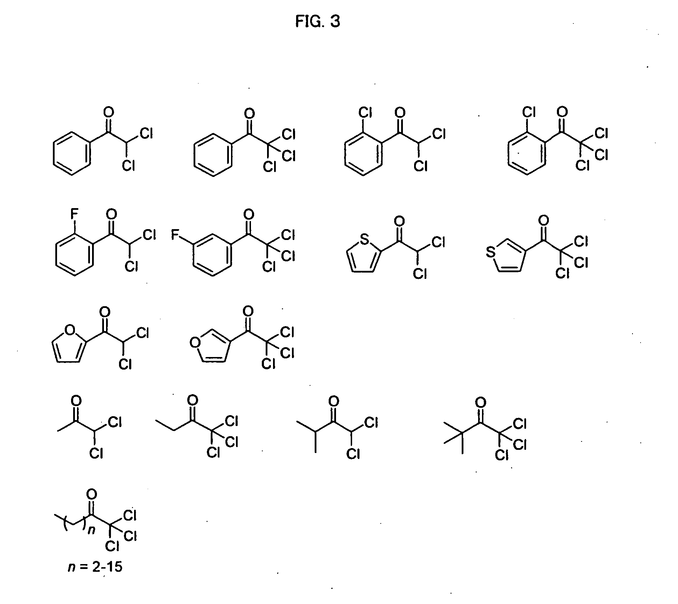

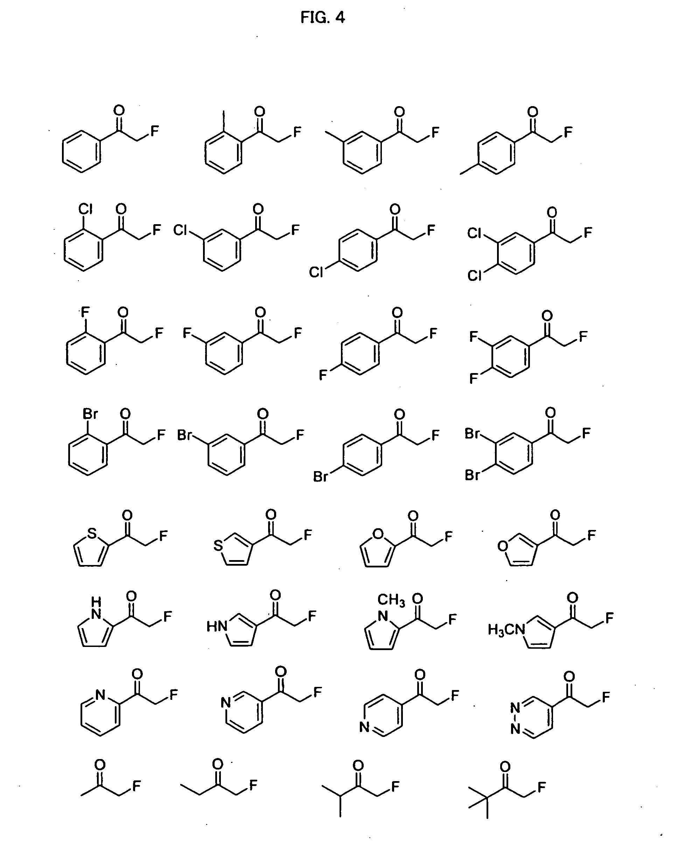

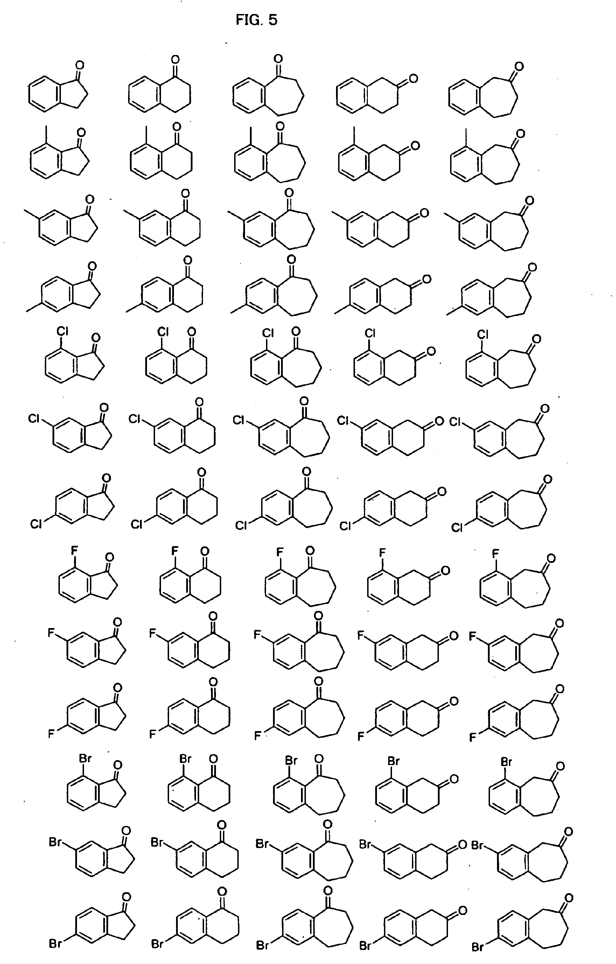

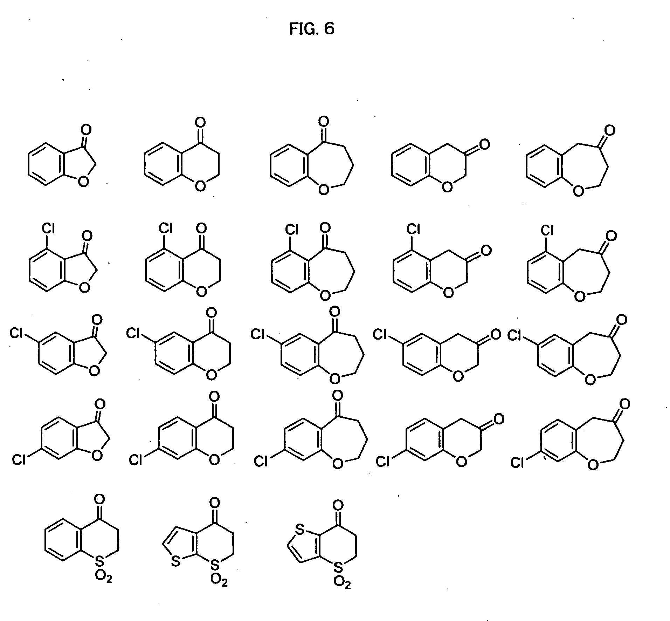

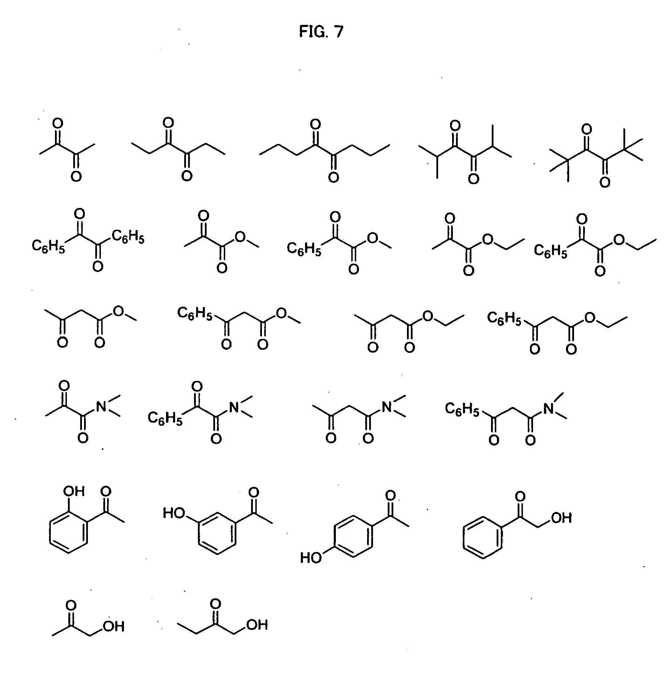

Fig. 1 is a first illustration that shows the structures of ketone compounds to which the process of producing an optically active alcohol of the present invention is applicable; Fig. 2 is a second illustration that also shows structures of ketone compounds; Fig. 3 is a third illustration that also shows structures of ketone compounds; Fig. 4 is a fourth illustration that also shows structures of ketone compounds; Fig. 5 is a fifth illustration that also shows structures of ketone compounds; Fig. 6 is a sixth illustration that also shows structures of ketone compounds; and Fig. 7 is a seventh illustration that also shows structures of ketone compounds.

### EXAMPLES

Hydrogenation of a carbonyl compound of the present invention may be conducted in a batch or continuous-flow system. Examples are described below to further describe the present invention in detail. It is to be understood that the present invention is not limited by the examples described below.

In the examples described below, the solvent used for the reaction was dried and degassed. For NMR measurements, JNM-LA400 (400 MHz, produced by JEOL Ltd.) and JNM-LA500 (500 MHz, produced by JEOL Ltd.) were used. For ¹H-NMR, tetramethylsilane (TMS) was used as the internal standard, and for ³¹P-NMR, 85% phosphoric acid was used as the external standard. For these signals, δ = 0 (δ denotes the chemical shift) was assumed. Optical purity was measured by gas chromatography (GC) or high-performance liquid chromatography (HPLC). GC measurement was conducted using Chirasil-DEX CB (0.25 mm × 25 m, DF = 0.25 µm) (produced by CHROMPACK), and HPLC measurement was conducted using a chiral compound isolation column (produced by Daicel). The metal complex represented by general formula (1) was synthesized by the technique disclosed in Angew. Chem., Int. Ed. Engl. Vol. 36, p. 285 (1997), and the metal complex represented by general formula (2) was synthesized by the technique disclosed in J. Org. Chem. Vol. 64, p. 2186 (1999).

### [EXAMPLE 1]

An example of synthesizing (S)-4-phenyl-3-butyn-2-ol by hydrogenation of 4-phenyl-3-butyn-2-one is described below. A 50 mL stainless steel autoclave was charged with a ruthenium complex, RuCl[(S,S)-Tsdpen](p-cymene), (1.6 mg, 0.0025 mmol), followed by argon substitution. 4-Phenyl-3-butyn-2-one (0.291 mL, 2 mmol) and methanol (5 mL) were added. After pressurization with hydrogen, substitution was conducted five times. Hydrogen was charged to 50 atm to initiate reaction. After the reaction mixture was stirred for 11 hours at 30°C, the reaction pressure was reduced to normal. The product was analyzed by ¹H-NMR and HPLC reporting synthesis of (S)-4-phenyl-3-butyn-2-ol in 90% ee and 63% yield. For the purpose of this description, in the nomenclature of the ruthenium complex, the metal atom, the anionic group, the diamine ligand, and the arene ligand are presented in this order from the left (see formula (4) below):

Formula (4)

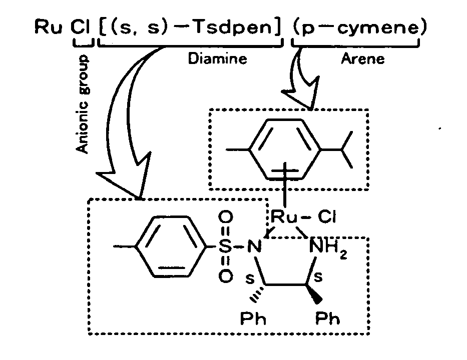

### [COMPARATIVE EXAMPLE 1]

Reaction was conducted under the same conditions as in EXAMPLE 1 but without pressurization with hydrogen. The target substance was not obtained.

### [EXAMPLES 2-10]

Reaction was conducted under the same conditions as in EXAMPLE 1 but with different catalysts and/or hydrogen pressures to synthesize (S)-4-phenyl-3-butyn-2-ol. The results are shown in Table 1. Table 1

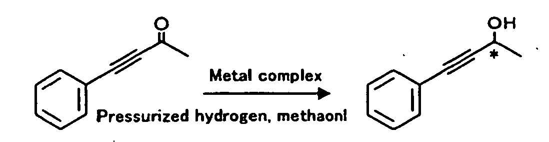

| Examples | chiral Ru cat | H₂ (atm) | yield (%) | ee (%) | config |
|---|---|---|---|---|---|
| 2 | RuCl[(*S,S*)-Tsdpen](p-cymene) | 9 | 18 | 81 | *S* |
| 3 | RuCl[(*S,S*)-Tsdpen](dmb) | 50 | 32 | 91 | *S* |
| 4 | RuCl[(*S,S*)-Tsdpen](mesitylene) | 50 | 100 | 79 | *S* |
| 5 | RuCl[(*S,S*)-Tsdpen](teb) | 50 | 61 | 91 | *S* |
| 6 | RuCl[(*S,S*)-Tsdpen](durene) | 50 | 29 | 71 | *S* |
| 7 | RuCl[(*S,S*)-Tsdpen](pmb) | 50 | 30 | 89 | *S* |
| 8 | RuCl[(*S,S*)-Tsdpen](hmb) | 50 | 78 | 88 | *S* |
| 9 | RuCl[(*S,S*)-Msdpen](p-cymene) | 50 | 78 | 88 | *S* |
| 10 | RuCl[(*S,S*)-(5,6,7,8-tetrahydronaphthalen e-2-yl)sulfonyl-dpen](*p*-cymene) | 50 | 69 | 91 | *S* |

| | | | | | |
|---|---|---|---|---|---|
| Conditions: chiral Ru cat 0.0025 mmol, CH₃OH 5 ml, SIC = 800, temp 30 °C, time 11 h, [ketone] = 0.4 M, dmb: 1,4-dimethylbenzene, teb: 1,3,5-triethylbenzene, durene: 1,2,4,5-tetramethylbenzene, pmb: pentamethylbenzene, hmb: hexamethylbenzene. | | | | | |

### [EXAMPLES 11-19]

Reaction was conducted under the same conditions as in EXAMPLE 1 but with different substrate concentrations, reaction temperature, and/or additives to synthesize (S)-4-phenyl-3-butyn-2-ol. The results are shown in Table 2. Table 2

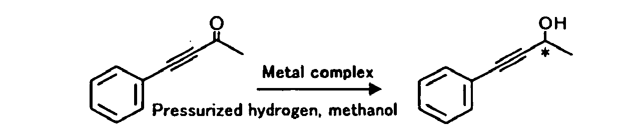

| Examples | additive | temp, °C | yield (%) | ee (%) | config |
|---|---|---|---|---|---|
| 11 | - | 50 | 50 | 87 | *S* |
| 12 *^{a}* | - | 30 | 27 | 75 | *S* |
| 13 *^{b}* | - | 30 | 33 | 88 | *S* |
| 14 | NaClO₄ 0.125 mmol | 30 | 88 | 92 | *S* |
| 15 | LiClO₄ 0.125 mmol | 30 | 80 | 92 | *S* |
| 16 | KClO₄ 0.125 mmol | 30 | 64 | 92 | *S* |
| 17 | BaClO₄ 0.125 mmol | 30 | 69 | 93 | *S* |
| 18 | NaPF₆ 0.125 mmol | 30 | 75 | 90 | *S* |
| 19 | NaBF₄ 0.125 mmol | 30 | 77 | 93 | *S* |

| | | | | | |
|---|---|---|---|---|---|
| Conditions: [ketone] = 0.4 M in CH₃OH, RuCl[(*S,S*)-Tsdpen](p-cymene) 0.0025 mmol, SIC = 800, H₂ 50 atm, time 11 h, solvent 5 ml, *^{a}* [ketone] = 0.1 M, *^{b}*[ketone] =1.0 M. | | | | | |

### [EXAMPLES 20-26]

Reaction was conducted under the same conditions as in EXAMPLE 1 but with different catalysts and solvents and use of additives to synthesize (S)-4-phenyl-3-butyn-2-ol. The results are shown in Table 3. Table 3

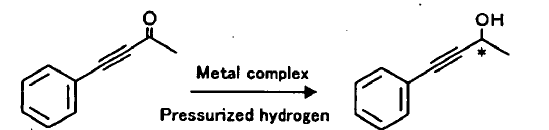

| Examples | chiral Ru cat | solvent | additive | yield (%) | ee (%) | config |
|---|---|---|---|---|---|---|
| 20 | RuCl[(*S*,*S*)-Tsdpen](p-cymene) | CH₃OH:H₂O = 99:1 | NaClO₄ 0.125 mmol | 79 | 89 | *S* |
| 21 | RuCl[(*S*,*S*)-Tsdpen](p-cymene) | CH₃OH:THF = 80:20 | NaClO₄ 0.125 mmol | 53 | 93 | *S* |
| 22 | RuCl[(*S*,*S*)-Tsdpen](p-cymene) | DMF:H₂O = 80:20 | NaClO₄ 0.125 mmol | 37 | 92 | *S* |
| 23 | RuCl[(*S*,*S*)-Tsdpen](p-cymene) | CH₃OH | NaClO₄ 0.025 mmol | 68 | 92 | *S* |
| 24 | RuCl[(*S*,*S*)-Tsdpen](p-cymene) | CH₃OH | NaClO₄ 2.5 mmol | 64 | 92 | *S* |
| 25 | RuCl[(*S*,*S*)-Tsdpen](mesitylene) | ^{a} CH₃OH | NaClO₄ 0.125 mmol | 69 | 90 | *S* |
| 26 | RuCl[(*S,S*)-Tsdpen](mesitylene) | ^{b} CH₃OH | NaClO₄ 0.125 mmol | 90 | 94 | *S* |

| | | | | | | |
|---|---|---|---|---|---|---|
| Conditions: [ketone] = 0.4 M, chiral Ru cat 0.0025 mmol, S/C = 800, H₂ 50 atm, temp 30 °C, time 11 h, solvent 5 ml, *^{a}* S/C = 2000. *^{b}* S/C = 2000, H₂ 100 atm. | | | | | | |

### [EXAMPLE 27]

An example of synthesizing (S)-indanol by hydrogenation of 1-indanone is described below. A 50 mL stainless steel autoclave was charged with a ruthenium complex, RuCl[(S,S)-Tsdpen](p-cymene), (1.6 mg, 0.0025 mmol) and 1-indanone (330 mg, 2. 5 mmol), followed by argon substitution. Methanol (5 mL) was added. Hydrogen was pressurized and substitution was conducted five times. Hydrogen was charged to 50 atm to initiate reaction. After the reaction mixture was stirred for 11 hours at 30°C, the reaction pressure was reduced to normal. The product was analyzed by ¹H-NMR and HPLC reporting synthesis of (S)-indanol in 98% ee and 48% yield.

### [EXAMPLES 28-31]

Reaction was conducted under the same conditions as in EXAMPLE 27 but with different catalysts, solvents, hydrogen pressures, and reaction times, and use of additives to synthesize an optically active indanol. The results are shown in Table 4. Table 4

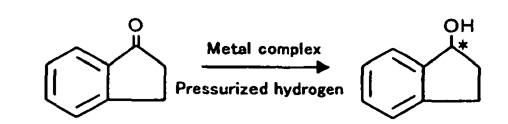

| Examples | chiral Ru cat | solvent | H₂ (atm) | yield (%) | ee (%) | config |
|---|---|---|---|---|---|---|
| 28 | RuCl[(*S,S*)-Tsdpen](mesitylene) | CH₃OH | 50 | 89 | 98 | *S* |
| 29 | RuCl[(*S,S*)-Tsdpen](mesitylene) | C₂H₅OH | 50 | 20 | 76 | *S* |
| 30 | RuCl(*S,S*)-Tsdpen](mesitylene) | CH₃OH | 100 | 86 | 98 | *S* |
| 31 | RuCl[(*S,S*)-Tsdpen](mesitylene) | *^{a}* CH₃OH | 50 | 98 | 98 | *S* |

| | | | | | | |
|---|---|---|---|---|---|---|
| Conditions: chiral Ru cat 0.0025 mmol, solvent 5 ml, S/C = 1000, temp 30 °C, time 11 h, [ketone]=0.5M. *^{a}*24h. | | | | | | |

### [EXAMPLE 32]

An example of synthesizing optically active 2-chloro-1-phenylethanol by hydrogenation of α-chloroacetophenone is described below. A 50 mL stainless steel autoclave was charged with a ruthenium complex, RuCl[(S,S)-Tsdpen](mesitylene) (1 mg, 0.0016 mmol) and α-chloroacetophenone (247 mg, 1.6 mmol). After argon substitution, methanol (3.2 mL) was added. Hydrogen was pressurized and substitution was conducted five times. Hydrogen was charged to 50 atm to initiate reaction. After stirring for 24 hours at 30°C, the reaction pressure was reduced to normal. The product was analyzed by ¹H-NMR and GC reporting synthesis of (R)-2-chloro-1-phenylethanol in 98% ee and 100% yield.

### [EXAMPLE 33]

An example of synthesizing optically active 2-chloro-1-phenylethanol by hydrogenation of α-chloroacetophenone is described below. A 50 mL stainless steel autoclave was charged with a ruthenium complex, RuCl[(S,S)-Tsdpen](mesitylene) (1 mg, 0.0016 mmol), and α-chloroacetophenone (1235 mg, 8.0 mmol). After argon substitution, methanol (16.0 mL) was added. Hydrogen was pressurized and substitution was conducted five times. Hydrogen was charged to 100 atm to initiate reaction. After stirring for 22 hours at 30°C, the reaction pressure was reduced to normal. The product was analyzed by ¹H-NMR and GC reporting synthesis of (R)-2-chloro-1-phenylethanol in 97% ee and 85% yield.

### [EXAMPLE 34-40]

Reaction was conducted under the same conditions as in EXAMPLE 32 but with different catalysts, hydrogen pressures, and reaction times to synthesize (R)-2-chloro-1 phenylethanol. The results are shown in Table 5. Table 5

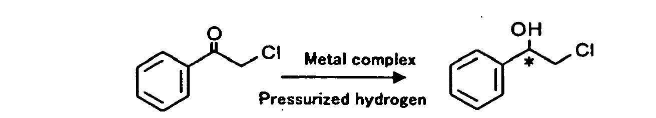

| Examples | chiral Ru cat | S/C | H₂ (atm) | yield (%) | ee (%) | config |
|---|---|---|---|---|---|---|
| 34 | RuCl[(*S*,*S*)-Tsdpen)(p-cymene) | 1000 | 50 | 48 | 92 | *R* |
| 35 | RuCl[(*S*,*S*)-Tsdpen](mesitylene) | 1500 | 50 | 100 | 98 | *R* |
| 36 | RuCl[(*S*,*S*)-Tsdpen](mesitylene) | 2000 | 50 | 88 | 98 | *R* |
| 37 | RuCl[(*S*,*S*)-Tsdpen](mesitylene) | 2000 | 100 | 100 | 98 | *R* |
| 38 | RuCl[(*S,S*)-Tsdpen](mesitylene) | 3000 | 100 | 100 | 97 | *R* |
| 39 | RuCl[(*S*,*S*)-Tsdpen](mesitylene) | 4000 | 100 | 96 | 98 | *R* |
| 40 | RuCl[(*S*,*S*)-Tsdpen](mesitylene)*^{a}* | 5000 | 50 | 46 | 97 | *R* |

| | | | | | | |
|---|---|---|---|---|---|---|
| Conditions: chiral Ru cat 0.0016 mmol, solvent CH₃OH, temp 30 °C, time 24 h, [ketone] = 0.5 M. *^{a}* 15 h. | | | | | | |

### [EXAMPLE 41]

An example of synthesizing optically active 2-chloro-1-phenylethanol by hydrogenation of α-chloroacetophenone is described below. Reaction was conducted under the same conditions as those of EXAMPLE 32 except that the reaction was conducted in the presence of a catalyst prepared from a ruthenium complex, Ru[(S,S)-Tsdpen](p-cymene), and HBF₄ and in a methanol/tert-butyl alcohol (1:1) mixture under a hydrogen pressure of 50 atm. As a result, (R)-2-chloro-1-phenylethanol was obtained in 95% ee and 100% yield.

### [EXAMPLE 42]

An example of synthesizing optically active 2-chloro-1-phenylethanol by hydrogenation of α-chloroacetophenone is described below. Reaction was conducted under the same conditions as those of EXAMPLE 32 except that rhodium complex CpRhCl[(S,S)-Tsdpen] (Cp: pentamethylcyclopentadiene) was used as a catalyst and the reaction was conducted for 11 hours. As a result, (R)-2-chloro-1-phenylethanol was obtained in 93% ee and 44% yield. Note that in the nomenclature of this rhodium complex, the cyclopentadiene ligand, the metal atom, the anionic group, and the diamine ligand are presented in this order from the left (see formula (5) below): Formula (5)

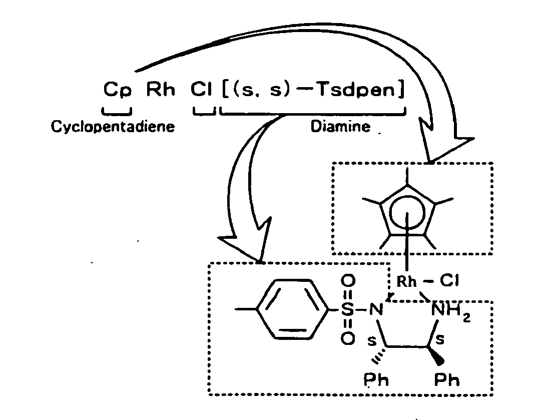

### [EXAMPLE 43]

An example of synthesizing optically active 2-chloro-1-(p-methoxyphenyl)ethanol by hydrogenation of α-chloro-p-methoxyacetophenone is described below. A 50 mL stainless-steel autoclave was charged with a ruthenium complex, RuCl[(S,S)-Tsdpen](mesitylene) (1 mg, 0.0016 mmol), α-chloro-p-methoxyacetophenone (1477 mg, 8.0 mmol), and NaClO₄

(10 mg, 0.08 mmol). After argon substitution, methanol (16.0 mL) was added. Hydrogen was pressurized, and substitution was conducted five times. Hydrogen was charged to 100 atm to initiate reaction. After stirring for 24 hours at 30°C, the reaction pressure was reduced to normal. The product was analyzed by ¹H-NMR and GC reporting synthesis of (R)-2-chloro-1-(p-methoxyphenyl)ethanol in 98% ee and 93% yield.

### [EXAMPLE 44]

An example of synthesizing optically active 2-chloro-1-(p-methoxyphenyl)ethanol by hydrogenation of α-chloro-p-methoxyacetophenone is described below. A 50 mL stainless steel autoclave was charged with a ruthenium complex, RuCl[(S,S)-Tsdpen](mesitylene) (1 mg, 0.0016 mmol), α-chloro-p-chloroacetophenone (605 mg, 3.2 mmol), and NaClO₄ (10 mg, 0.08 mmol). After argon substitution, methanol (6.4 mL) was added. Hydrogen was pressurized, and substitution was conducted five times. Hydrogen was charged to 100 atm to initiate reaction. After stirring for 24 hours at 30°C, the reaction pressure was reduced to normal. The product was analyzed by ¹H-NMR and GC reporting synthesis of (R)-2-chloro-1-(p-chlorophenyl)ethanol in 95% ee and 93% yield.

### [EXAMPLE 45]

An example of synthesizing optically active 4-chromanol by hydrogenation of chromanone is described below. A 50 mL stainless steel autoclave was charged with RuCl[(S,S)-Tsdpen](mesitylene) (1.0 mg, 0.0016 mmol) under argon. Then 4-chromanone (474 mg, 3.2 mmol) and methanol (6.4 mL) were added thereto. After pressurization with hydrogen, substitution was conducted five times. Hydrogen was charged to 50 atm to initiate reaction. After stirring for 23 hours at 30°C, the reaction pressure was reduced to normal. The product was analyzed by ¹H-NMR and HPLC reporting synthesis of (S)-4-chromanol in 91% ee and 100% yield.

### [EXAMPLE 46]

An example of synthesizing optically active 4-chromanol by hydrogenation of chromanone is described below. A 50 mL stainless steel autoclave was charged with Rucl[(S,S)-Tsdpen](p-cymene) (1.0 mg, 0.0016 mmol) under argon. Then 4-chromanone (474 mg, 3.2 mmol) and methanol (6.4 mL ) were added thereto. After pressurization with hydrogen, substitution was conducted five times. Hydrogen was charged to 50 atm to initiate reaction. After stirring for 23 hours at 30°C, the reaction pressure was reduced to normal. The product was analyzed by ¹H-NMR and HPLC reporting synthesis of (S)-4-chromanol in 97% ee and 85% yield.

### [EXAMPLE 47]

An example of synthesizing optically active 4-chromanol by hydrogenation of chromanone is described below. A 50 mL stainless steel autoclave was charged with RuCl[(S,S)-Tsdpen](p-cymene) (1.0 mg, 0.0016 mmol) and NaClO₄ (10 mg, 0.08 mmol) under argon. Then 4-chromanone (1185 mg, 8.0 mmol) and methanol (16 mL) were added thereto. After pressurization with hydrogen, substitution was conducted five times. Hydrogen was charged to 50 atm to initiate reaction. After stirring for 23 hours at 30°C, the reaction pressure was reduced to normal. The product was analyzed by ¹H-NMR and HPLC reporting synthesis of (S)-4-chromanol in 97% ee and 93% yield.

### [EXAMPLE 48]

An example of synthesizing optically active (3'-hydroxyphenyl)ethanol by hydrogenation of 3'-hydroxyacetophenone is described below. A 50 mL stainless steel autoclave was charged with RuCl[(S,S)-Tsdpen](mesitylene) (0.93 mg, 0.0015 mmol) and NaClO₄ (9.2 mg, 0.075 mmol) under argon. Then 3'-hydroxyacetophenone (613 mg, 4.5 mmol) and methanol (9 mL) were added thereto. After pressurization with hydrogen, substitution was conducted five times. Hydrogen was charged to 100 atm to initiate reaction. After stirring for 20 hours at 30°C, the reaction pressure was reduced to normal. The product was analyzed by ¹H-NMR and HPLC reporting synthesis of optically active (3'-hydroxyphenyl)ethanol in 98% ee and 98% yield.

### [EXAMPLE 49]

An example of synthesizing optically active 5,6-dihydro-4H-thieno[2,3-b]thiopyran-4-hydroxy-7,7-dioxid e by hydrogenation of 5,6-dihydro-4H-thieno[2,3-b]thiopyran-4-one-7,7-dioxide is described below. A 50 mL stainless steel autoclave was charged with RuCl[(S,S)-Tsdpen](mesitylene) (0.93 mg, 0.0015 mmol) and NaClO₄ (9.2 mg, 0.075 mmol) under argon. Then 5,6-dihydro-4H-thieno[2,3-b]thiopyran-4-one-7,7-dioxide (455 mg, 2.25 mmol) and methanol (22.5 mL) were added thereto. After pressurization with hydrogen, substitution was conducted five times. Hydrogen was charged to 100 atm to initiate reaction. After stirring for 24 hours at 30°C, the reaction pressure was reduced to normal. The product was analyzed by ¹H-NMR and HPLC reporting synthesis of (S)-5,6-dihydro-4H-thieno[2,3-b]thiopyran-4-hydroxy-7,7-di oxide in 98% ee and 100% yield.

### [EXAMPLE 50]

An example of synthesizing optically active 1,2-propanediol by hydrogenation of acetol is described below. A 50 mL stainless steel autoclave was charged with RuCl[(S,S)-Tsdpen](mesitylene) (0.93 mg, 0.0015 mmol) and NaClO₄ (9.2 mg, 0.075 mmol) under argon. Then acetol (111 mg, 1.5 mmol) and methanol (3.0 mL) were added thereto. After pressurization with hydrogen, substitution was conducted five times. Hydrogen was charged to 100 atm to initiate reaction. After stirring for 17 hours at 30°C, the reaction pressure was reduced to normal. The product was analyzed by ¹H-NMR and HPLC reporting synthesis of (R)-1,2-propanediol in 63% ee and 97% yield.

### [EXAMPLE 51]

An example of synthesizing optically active 2,3-butanediol by hydrogenation of 2,3-butanedione is described below. A 50 mL stainless steel autoclave was charged with RuCl[(S,S)-Tsdpen](p-cymene) (0.95 mg, 0.0015 mmol) and NaClO₄ (9.2 mg, 0.075 mmol) under argon. Then 2,3-butanedione (129 mg, 1.5 mmol) and methanol (3.0 mL) were added thereto. After pressurization with hydrogen, substitution was conducted five times. Hydrogen was charged to 50 atm to initiate reaction. After stirring for 18 hours at 30°C, the reaction pressure was reduced to normal. The product was analyzed by ¹H-NMR and HPLC reporting synthesis of (S,S)-2,3-butanediol in yield of 47%.

The following Examples 52-54 and Comparative Examples 2-4 do not fall within the scope of the invention as claimed.

### [EXAMPLE 52]

An example of synthesizing (R)-4-phenyl-3-butyn-2-ol by hydrogenation of 4-phenyl-3-butyn-2-one is described below. A 50 mL stainless steel autoclave was charged with a ruthenium complex, RuHCl[(S)-tolbinap][(S,S)-dpen] (1 mg, 0.00097 mmol). After argon substitution, 4-phenyl-3-butyn-2-one (0.283 mL, 1.94 mmol) and methanol (1.9 mL) were added thereto. Hydrogen was pressurized and substitution was conducted (five times). Hydrogen was charged to 9 atm to initiate reaction. After stirring for 11 hours at 30°C, the reaction pressure was reduced to normal, and the reaction solution was analyzed by ¹H-NMR and HPLC to determine the determinate quantity and optical purity of the product, i.e., 4-phenyl-3-butyn-2-ol. As a result, (R)-4-phenyl-3-butyn-2-ol was obtained in 74% ee and 65% yield. In the nomenclature of this ruthenium complex, the metal atom, the hydrogen atom, the anionic group, the diphosphine ligand, and the diamine ligand are presented in this order from the left (see formula (6) below). Formula (6)

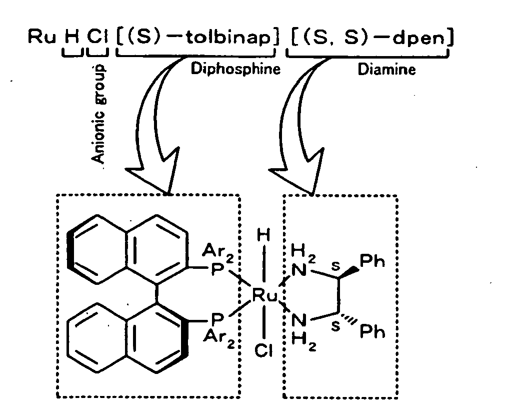

### [EXAMPLES 53-54]

Reaction was conducted as in EXAMPLE 52 but with a ruthenium complex, RuHCl[(S,S)-tolbinap][(S,S)-dpen] as a catalyst and with different reaction temperatures and additives. The results are shown in Table 6. Table 6

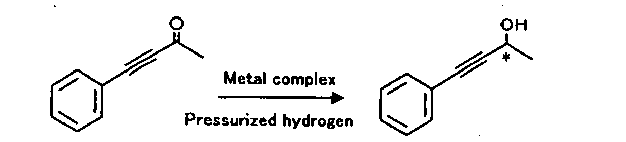

| Examples | chiral Ru cat | temp, °C | additive | yield (%) | ee (%) | config |
|---|---|---|---|---|---|---|
| 53 | RuHCl[(S)-tolbinapl[(S,S)-dpen] | 50 | - | 100 | 75 | *R* |
| 54 | RuHCl[(S)-tolbinap][(S,S)-dpen] | 30 | NaClO₄ 0.05 mol | 96 | 75 | *R* |
| 2^{a} | RuH(BH₄)[(S)-tolbinap][(S,S)-dpen] | 30 | NaClO₄ 0.05 mol | 20 | 76 | *R* |

| | | | | | | |
|---|---|---|---|---|---|---|
| Conditions: chiral Ru cat 0.001 mmol, CH₃OH 2 ml, S/C = 2000, time 11 h, H₂ 9 atm, [ketone] = 1.0 M. ^{a} Comparative example | | | | | | |

### [COMPARATIVE EXAMPLE 2]

Reaction was conducted as in EXAMPLE 52 but with a ruthenium complex, RuH (BH4) [(S,S)-tolbinap][(S,S)-dpen], as a catalyst and with different reaction temperature and additive. The results are shown in Table 6.

### [COMPARATIVE EXAMPLE 3]

A 50 mL stainless steel autoclave was charged with a ruthenium complex, RuCl₂[(S)-tolbinap][(S,S)-dpen] (1 mg, 0.00097 mmol) and KOt-Bu (0.1 mg, 0.00097 mmol). After argon substitution, 4-phenyl-3-butyn-2-one (0.283 mL, 1.94 mmol) and methanol (1.9 mL) were added thereto. Hydrogen was pressurized and substitution was conducted (five times). Hydrogen was charged to 9 atm to initiate reaction. After stirring for 11 hours at 30°C, the reaction pressure was reduced to normal, and the reaction solution was analyzed by ¹H-NMR, reporting generation of only trace amounts of 4-phenyl-3-butyn-2-ol.

### [COMPARATIVE EXAMPLE 4]

Reaction of 4-phenyl-3-butyn-2-one was conducted as in COMPARATIVE EXAMPLE 2 but in 2-propanol. The reaction solution was analyzed by ¹H-NMR, reporting generation of only trace amounts of 4-phenyl-3-butyn-2-ol.

### Industrial Applicability

The present invention is applicable to production of an optically active alcohol usable as an intermediate or the like of medicines, agricultural chemicals, and many general-purpose chemical agents.

## Claims

1. A process for producing an optically active alcohol, comprising placing a metal complex represented by general formula (1) below and a ketone compound in a polar solvent and stirring the mixture under pressurized hydrogen to hydrogenate the ketone compound to thereby obtain the optically active alcohol:
General Formula (1) 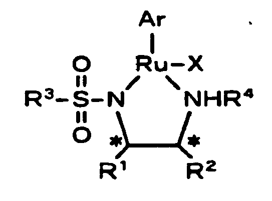 (where R¹ and R² may be the same or different and are each selected from the group consisting of an alkyl group, an optionally substituted phenyl group, an optionally substituted naphthyl group, and an optionally substituted cycloalkyl group, or together form an optionally substituted alicyclic ring;
R³ is one selected from the group consisting of an alkyl group, a perfluoroalkyl group, an optionally substituted naphthyl group, an optionally substituted phenyl group, and a camphor group;
R⁴ is a hydrogen atom or an alkyl group;
Ar is an optionally substituted benzene;
X is an anionic group; and
* represents an asymmetric carbon.)

2. A process for producing an optically active alcohol, comprising placing a metal complex represented by general formula (2) and a ketone compound in a polar solvent and stirring the mixture under pressurized hydrogen to hydrogenate the ketone compound to thereby obtain the optically active alcohol:
General Formula (2) 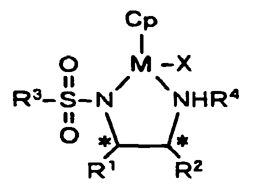 (where R¹ and R² may be the same or different and are each selected from the group consisting of an alkyl group, an optionally substituted phenyl group, an optionally substituted naphthyl group, and an optionally substituted cycloalkyl group, or together form an optionally substituted alicyclic ring;
R³ is one selected from the group consisting of an alkyl group, a perfluoroalkyl group, an optionally substituted naphthyl group, an optionally substituted phenyl group, and a camphor group;
R⁴ is a hydrogen atom or an alkyl group;
Cp is an optionally substituted cyclopentadiene;
M is rhodium or iridium;
X is an anionic group; and
* represents an asymmetric carbon.)

3. The process for producing the optically active alcohol according to claim 1 or 2, wherein in general formulae (1) and (2), R¹, R², and R³ may be the same or different and each represent a phenyl group, a phenyl group having a C₁-C₅ alkyl group, a phenyl group having a C₁-C₅ alkoxy group, or a phenyl group having a halogen substituent.

4. The process for producing the optically active alcohol according to any one of claims 1 to 3, wherein the polar solvent is methanol or ethanol.

5. The process for producing the optically active alcohol according to any one of claims 1 to 4, wherein no base is added.

6. The process for producing the optically active alcohol according to any one of claims 1 to 5, wherein the ketone compound is unstable in the presence of bases.

7. The process for producing the optically active alcohol according to any one of claims 1 to 6, wherein the ketone compound is a cyclic ketone, a ketone having an olefin moiety, a ketone having an acetylene moiety, a ketone having a hydroxyl group, a ketone having a halogen substituent, a chromanone derivative, a diketone, a ketoester, or a ketoamide.

8. The process for producing the optically active alcohol according to any one of claims 1 to 7, wherein the ketone compound is a ketone compound having a halogen substituent at α-position or α,β-alkynyl ketone.

## Patentansprüche

1. Verfahren zur Herstellung eines optisch aktiven Alkohols, umfassend das Bringen eines Metallkomplexes der unten dargestellten allgemeinen Formel (1) und einer Ketoverbindung in ein polares Lösungsmittel und das Rühren des Gemisches unter Wasserstoff unter Druck, um die Ketoverbindung zu hydrieren, um **dadurch** den optisch aktiven Alkohol zu erhalten:
Allgemeine Formel (1) 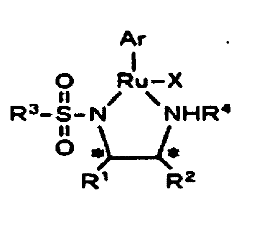 (wobei R¹ und R² für dieselbe oder verschiedene Gruppen stehen können und jeweils aus der Gruppe ausgewählt sind, die aus einer Alkylgruppe, einer gegebenenfalls substituierten Phenylgruppe, einer gegebenenfalls substituierten Naphthylgruppe und einer gegebenenfalls substituierten Cycloalkylgruppe besteht, oder zusammen einen gegebenenfalls substituierten alicyclischen Ring bilden können;
R³ für eine Gruppe steht, die aus der Gruppe ausgewählt ist, die aus einer Alkylgruppe, einer Perfluoralkylgruppe, einer gegebenenfalls substituierten Naphthylgruppe, einer gegebenenfalls substituierten Phenylgruppe und einer Camphergruppe besteht;
R⁴ für ein Wasserstoffatom oder eine Alkylgruppe steht;
Ar für einen gegebenenfalls substituierten Benzolring steht;
X für eine anionische Gruppe steht und
* einen asymmetrischen Kohlenstoff kennzeichnet.)

2. Verfahren zur Herstellung eines optisch aktiven Alkohols, umfassend das Bringen eines Metallkomplexes der unten dargestellten allgemeinen Formel (2) und einer Ketoverbindung in ein polares Lösungsmittel und das Rühren des Gemisches unter Wasserstoff unter Druck, um die Ketoverbindung zu hydrieren, um **dadurch** den optisch aktiven Alkohol zu erhalten:
Allgemeine Formel (2) 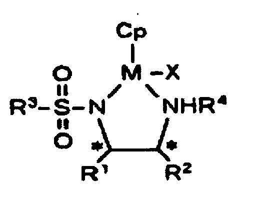 (wobei R¹ und R² für dieselbe oder verschiedene Gruppen stehen können und jeweils aus der Gruppe ausgewählt sind, die aus einer Alkylgruppe, einer gegebenenfalls substituierten Phenylgruppe, einer gegebenenfalls substituierten Naphthylgruppe und einer gegebenenfalls substituierten Cycloalkylgruppe besteht, oder zusammen einen gegebenenfalls substituierten alicyclischen Ring bilden können;
R³ für eine Gruppe steht, die aus der Gruppe ausgewählt ist, die aus einer Alkylgruppe, einer Perfluoralkylgruppe, einer gegebenenfalls substituierten Naphthylgruppe, einer gegebenenfalls substituierten Phenylgruppe und einer Camphergruppe besteht;
R⁴ für ein Wasserstoffatom oder eine Alkylgruppe steht;
Cp für einen gegebenenfalls substituierten Cyclopentadienring steht;
M für Rhodium oder Iridium steht;
X für eine anionische Gruppe steht und
* einen asymmetrischen Kohlenstoff kennzeichnet.)

3. Verfahren zur Herstellung eines optisch aktiven Alkohols nach Anspruch 1 oder 2, wobei in den allgemeinen Formeln (1) und (2) R¹, R² und R³ für dieselben oder verschiedene Gruppen stehen können und jeweils für eine Phenylgruppe, eine Phenylgruppe mit einer C₁₋₅-Alkylgruppe, eine Phenylgruppe mit einer C₁₋₅-Alkoxygruppe oder eine Phenylgruppe mit einem Halogensubstituenten stehen können.

4. Verfahren zur Herstellung des optisch aktiven Alkohols nach einem der Ansprüche 1 bis 3, wobei es sich bei dem polaren Lösungsmittel um Methanol oder Ethanol handelt.

5. Verfahren zur Herstellung des optisch aktiven Alkohols nach einem der Ansprüche 1 bis 4, wobei keine Base hinzu gegeben wird.

6. Verfahren zur Herstellung des optisch aktiven Alkohols nach einem der Ansprüche 1 bis 5, wobei die Ketoverbindung in Gegenwart von Basen instabil ist.

7. Verfahren zur Herstellung des optisch aktiven Alkohols nach einem der Ansprüche 1 bis 6, wobei es sich bei der Ketoverbindung um ein cyclisches Keton, ein Keton mit einer Olefineinheit, ein Keton mit einer Acetyleneinheit, ein Keton mit einer Hydroxylgruppe, ein Keton mit einem Halogensubstituenten, ein Chromanonderivat, ein Diketon, einen Ketoester oder ein Ketoamid handelt.

8. Verfahren zur Herstellung des optisch aktiven Alkohols nach einem der Ansprüche 1 bis 7, wobei es sich bei der Ketoverbindung um eine Ketoverbindung mit einem Halogensubstituenten in α-Position oder ein α,β-Alkinylketon handelt.

## Revendications

1. Procédé de production d'un alcool optiquement actif, qui comprend le fait de mettre un complexe métallique représenté par la formule générale (1) ci-dessous et un composé cétone dans un solvant polaire et le fait d'agiter le mélange sous hydrogène pressurisé pour hydrogéner le composé cétone et ainsi obtenir l'alcool optiquement actif :
Formule Générale (1) 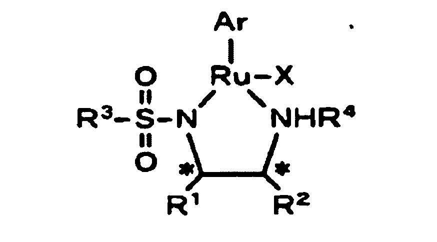 (dans laquelle R¹ et R² peuvent être identiques ou différents et sont chacun choisis dans le groupe constitué par un groupe alkyle, un groupe phényle facultativement substitué, un groupe naphtyle facultativement substitué, et un groupe cycloalkyle facultativement substitué, ou forment conjointement un cycle alicyclique facultativement substitué ;
R³ est choisi dans le groupe constitué par un groupe alkyle, un groupe perfluoroalkyle, un groupe naphtyle facultativement substitué, un groupe phényle facultativement substitué, et un groupe camphre ;
R⁴ est un atome d'hydrogène ou un groupe alkyle ;
Ar est un groupe benzène facultativement substitué ;
X est un groupe anionique ; et
* représente un atome de carbone asymétrique.)

2. Procédé de production d'un alcool optiquement actif, qui comprend le fait de mettre un complexe métallique représenté par la formule générale (2) et un composé cétone dans un solvant polaire et le fait d'agiter le mélange sous hydrogène pressurisé pour hydrogéner le composé cétone et ainsi obtenir l'alcool optiquement actif :
Formule Générale (2) 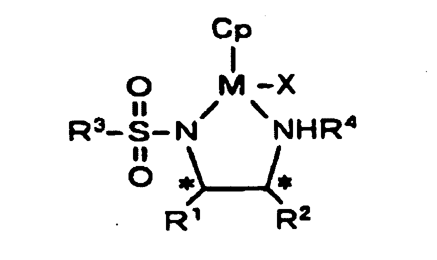 (dans laquelle R¹ et R² peuvent être identiques ou différents et sont chacun choisis dans le groupe constitué par un groupe alkyle, un groupe phényle facultativement substitué, un groupe naphtyle facultativement substitué, et un groupe cycloalkyle facultativement substitué, ou forment conjointement un cycle alicyclique facultativement substitué ;
R³ est choisi dans le groupe constitué par un groupe alkyle, un groupe perfluoroalkyle, un groupe naphtyle facultativement substitué, un groupe phényle facultativement substitué, et un groupe camphre ;
R⁴ est un atome d'hydrogène ou un groupe alkyle ;
Cp est un groupe cyclopentadiène facultativement substitué ;
M représente le rhodium ou l'iridium ;
X est un groupe anionique ; et
* représente un atome de carbone asymétrique.)

3. Procédé de production de l'alcool optiquement actif selon la revendication 1 ou 2, dans lequel dans les formules générales (1) et (2), R¹, R², et R³ peuvent être identiques ou différents et représentent chacun un groupe phényle, un groupe phényle ayant un groupe alkyle en C₁-C₅, un groupe phényle ayant un groupe alcoxy en C₁-C₅, ou un groupe phényle ayant un substituant halogène.

4. Procédé de production de l'alcool optiquement actif selon l'une quelconque des revendications 1 à 3, le solvant polaire étant le méthanol ou l'éthanol.

5. Procédé de production de l'alcool optiquement actif selon l'une quelconque des revendications 1 à 4, aucune base n'étant ajoutée:

6. Procédé de production de l'alcool optiquement actif selon l'une quelconque des revendications 1 à 5, le composé cétone étant instable en présence de bases.

7. Procédé de production de l'alcool optiquement actif selon l'une quelconque des revendications 1 à 6, le composé cétone étant une cétone cyclique, une cétone ayant une fraction oléfinique, une cétone ayant une fraction acétylène, une cétone ayant un groupe hydroxyle, une cétone ayant un substituant halogène, un dérivé du chromanone, une dicétone, un cétoester, ou un cétoamide.

8. Procédé de production de l'alcool optiquement actif selon l'une quelconque des revendications 1 à 7, le composé cétone étant un composé cétone ayant un substituant halogène en position α ou une α,β-alcynyl cétone.
